(19) Europäisches Patentamt
European Patent Office
Office européen des brevets

(11) Numéro de publication : 0 282 374 B1

# (12) FASCICULE DE BREVET EUROPEEN

(45) Date de publication du fascicule du brevet : **02.05.91 Bulletin 91/18**

(51) Int. Cl.$^5$ : **C07K 5/08,** C07K 5/10, A61K 37/02

(21) Numéro de dépôt : **88400315.3**

(22) Date de dépôt : **12.02.88**

(54) **Nouveaux dérivés peptidiques à structure polycyclique azotée, leur procédé de préparation et les compositions pharmaceutiques qui les contiennent.**

(30) Priorité : **13.02.87 FR 8701810**

(43) Date de publication de la demande : **14.09.88 Bulletin 88/37**

(45) Mention de la délivrance du brevet : **02.05.91 Bulletin 91/18**

(84) Etats contractants désignés : **AT BE CH DE FR GB IT LI LU NL SE**

(56) Documents cités :
**EP-A- 0 190 058**
**DE-A- 2 343 034**
**FR-A- 2 453 136**
**Int. J. Peptide, Protein Research, 1978, 12, 130-138**

(73) Titulaire : **ADIR ET COMPAGNIE**
**22, rue Garnier**
**F-92201 Neuilly sur Seine (FR)**

(72) Inventeur : **Vincent, Michel**
**8 allée du Prunier Hardy**
**F-92220 Bagneux (FR)**
Inventeur : **Remond, Georges**
**9 avenue des Etats-Unis**
**F-78000 Versailles (FR)**
Inventeur : **Cudennec, Claude**
**23 bis avenue de Circourt**
**F-78170 La Celle St-Cloud (FR)**

EP 0 282 374 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux tétrapeptides, leur préparation et les compositions pharmaceutiques qui les contiennent.

On connaît de nombreux tétrapeptides, naturels ou synthétiques et notamment la tuftsine (Thr - Lys - Pro - Arg) modificateurs de la réponse biologique. Certains analogues de la tuftsine, particulièrement des composés dans lesquels la thréonine est remplacée par un radical carboxy - 4 oxazolidinone - 2 ont été décrits par Y. STABINSKY et coll. in Int. J. Peptide, Protéin Research 1978 ; 12 ; 130-138. Toutefois, ces dérivés ne retiennent seulement que la moitié environ de l'activité de la tuftsine en n'ont qu'une faible activité sur la réponse anticorps.

La demande de brevet européen n° 0 190 058 décrit des analogues de la tuftsine dans lesquels la proline est remplacée par une structure polycyclique azotée. Ces dérivés, possèdent, en général, une activité supérieure à celle de la tuftsine.

La demanderesse a maintenant découvert des analogues de la tuftsine dans lesquels la proline est remplacée par une structure polycyclique azotée de même type que celle des dérivés de la demande de brevet européen 0 190 058 et le reste thréonyle est cyclisé. De façon surprenante, ces dérivés possèdent une activité supérieure à celle de la tuftsine, au moins équivalente à celle des dérivés de la demande 0 190 058 mais en outre, ils présentent l'avantage supplémentaire de favoriser très nettement la réponse anticorps. Cette caractéristique permet de supposer que les composés de la présente invention agissent par un mécanisme d'action différente de celui des composés de l'art antérieur, propriété particulièrement intéressante dans le domaine thérapeutique où ces dérivés sont utilisables.

Plus spécifiquement, l'invention concerne des dérivés tétrapeptidiques de formule générale :

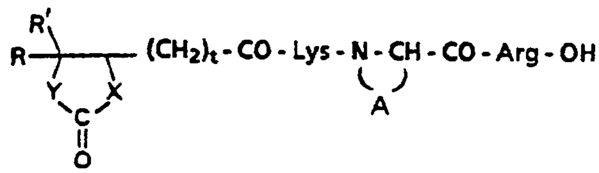

I

dans laquelle

R représente :

- un atome d'hydrogène
- un radical alcoyle comportant de 1 à 4 atomes de carbone, en chaîne droite ou ramifiée,
- un radical phényle ou thiényle éventuellement substitué par un groupement hydroxy, amino, mercapto, méthylthio, ou alcoyle inférieur,
- un radical benzyle ;

R' représente un atome d'hydrogène ou un groupement alcoyle comportant de 1 à 4 atomes de carbone en chaîne droite ou ramifiée,

X représente un atome d'oxygène ou un groupement NH,

Y représente un atome d'oxygène ou de soufre lorsque X représente un groupement NH ou Y représente un groupement NH lorsque X représente un atome d'oxygène,

t représente 0 ou 1,

Lys et Arg représentent respectivement les restes lysyle et arginyle engagés dans des liaisons peptidiques,

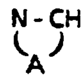

représente

1) une structure bicyclique de formule :

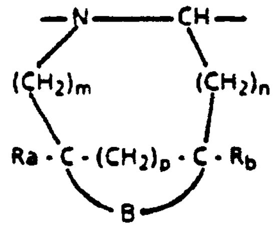

où

– m est égal à 1 ou zéro,

– n et p représentent zéro, 1 ou 2,

– $R_a$ et $R_b$ représentent un atome d'hydrogène ou peuvent former ensemble une liaison directe quand p = 0,

– B représente une chaîne alkylène $(CH_2)_q$ où q est égal à 2, 3 ou 4 ou bien une structure insaturée $(-CH = CH-)_2$ quand p = 0 et $R_a$ et $R_b$ forment ensemble une liaison avec la réserve que la somme de m, n, p et q est un nombre entier compris entre 3 et 6, ou

2) la tétrahydro-1, 2, 3, 4-bétacarboline, leurs énantiomères, épimères et diastéréoisomères, ainsi que leurs sels d'addition à un acide ou une base pharmaceutiquement acceptable.

Actuellement, parmi les composés de formule I, on préfère ceux dans lesquels la structure cyclique

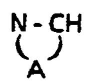

représente : l'indoline, l'isoindoline, la tétrahydroquinoléine, la tétrahydroisoquinoléine, le perhydroindole, le perhydroisoindole, la perhydroisoquinoléine, la perhydroquinoléine, le perhydrocyclopenta [b] pyrrole, l'aza - 2 - bicyclo [2.2.2] octane, l'aza - 2 bicyclo [2.2.1] heptane, la tétrahydro - 1, 2, 3, 4 bétacarboline.

Parmi les acides que l'on peut ajouter aux composés de formule I pour former un sel d'addition, on peut citer, à titre d'exemple, les acides chlorhydrique, sulfurique, phosphorique, tartrique, malique, maléique, fumarique, oxalique, méthane-sulfonique, éthanesulfonique, camphorique, citrique, etc...

Comme bases pouvant salifier les composés de formule I on pourra utiliser des hydroxydes de sodium, potassium, calcium ou d'aluminium, des carbonates de métaux alcalins ou alcalinoterreux ou des bases organiques comme la triéthylamine, la benzylamine, la diéthanolamine, la tert. butylamine, la dicyclohexylamine, l'arginine, etc...

L'invention s'étend aussi au procédé d'obtention des composés de formulé I, caractérisé en ce que l'on condense un dérivé de formule II :

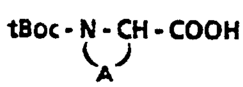

II

obtenu comme décrit dans la demande de brevet européen n° 0 190 058, dans lequel tBoc représente le radical tertiobutoxycarbonyle et A avec les atomes de carbone et d'azote auxquels il est attaché, a les mêmes significations que dans la formule I, avec $N_\omega$ - nitroarginate de méthyle (H - Arg $(NO_2)$ $OCH_3$) ou de benzyle (H - Arg $(NO_2)$ $OCH_2$ $C_6H_5$) pour obtenir un dérivé de formule III :

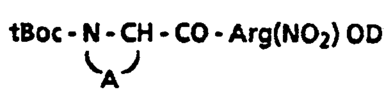

III

dans lequel A, avec les atomes de carbone et d'azote auxquels il est attaché, a les mêmes significations que

dans la formule I, et dans lequel D représente un radical méthyle ou benzyle, que l'on déprotège ensuite par l'acide trifluoroacétique selon la méthode décrit par B. GUTTE et K.B. MERRIFIELD (J. Am. Chem. Soc 1969, 91, 501), en un dérivé de formule IV :

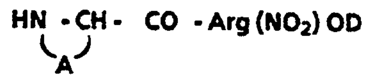

IV

dans lequel A, avec les atomes de carbone et d'azote auxquels il est attaché, a les mêmes significations que dans la formule I, et dans lequel D a la même signification que dans la formule III, qui est ensuite condensé avec la $N_\alpha$ - tertiobutoxycarbonyl $N_\omega$ benzyloxycarbonyl lysine ou (tBoc) Lys (Z) pour obtenir un composé de formule V :

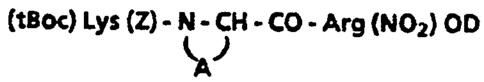

V

dans lequel A, avec les atomes de carbone et d'azote auxquels il est attaché, a les mêmes significations que dans la formule I et D la même signification que dans la formule III, qui est soumis à l'action de l'acide trifluoroacétique et converti en un dérivé de formule VI :

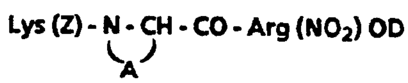

VI

dans lequel A, avec les atomes de carbone et d'azote auxquels il est attaché, a les mêmes significations que dans la formule I et D la même signification dans la formule III, que l'on condense avec un dérivé de formule VII :

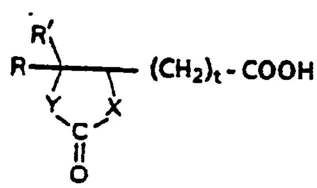

VII

dans lequel

R, R', X, Y et t ont la même signification que dans la formule I pour conduire à un dérivé de formule VIII :

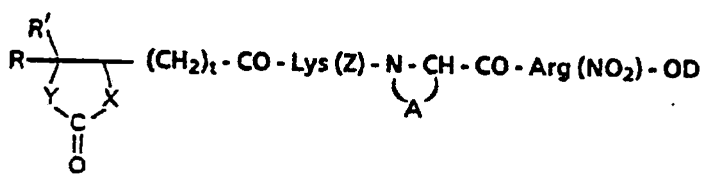

VIII

dans lequel A, avec les atomes de carbone et d'azote auxquels il est attaché, R, R', X, Y et t ont la même signification que dans la formule I et D la même signification que dans la formule III qui peut être soit :

– quand D représente $CH_3$, déprotégé par saponification puis soumis à hydrogénation catalytique ;
– quand D représente le groupement benzyle soumis à hydrogénation catalytique pour conduire à un dérivé de formule I que l'on peut si l'on désire :
– soit salifier par un acide ou une base pharmaceutiquement acceptable.
– soit séparer en ses isomères, puis, si nécessaire, salifier par un acide ou une base pharmaceutiquement

acceptable.

Les dérivés de formule VIII sont nouveaux et font partie de l'invention au même titre que les dérivés de formule I, dont ils constituent les intermédiaires de synthèse.

Les composés de formule I sont doués de propriétés pharmacologiques intéressantes.

En particulier, on retrouve, chez ces dérivés, à un niveau supérieur ou, au moins comparable, les principales propriétés des composés de la demande de brevet européen 0190 058.

Notamment, ces dérivés augmentent l'activité des cellules N.K. "Tueuses spontanées". Administrés à la souris porteuse d'un mélanome, ils inhibent de façon importante la croissance de ce mélanome. Ils assurent une promotion des défenses immunitaires chez des animaux infectés par des souches bactériennes pathogènes et, de façon inattendue par rapport à l'état de l'art, augmentent de façon importante la réponse anticorps aux antigènes de mouton chez la souris, la phagocytose non spécifique et l'hypersensibilité retardée à l'oxazolone.

Ces acitivités sont liées aux propriétés immunomodulatrices des composés de l'invention qui trouvent leur application, en thérapeutique animale ou humaine, dans le traitement des cancers, des affections d'origine virale, bactérienne ou fongique, des maladies auto-immunes comme le lupus érythémateux ou l'arthrite rhumatoïde et plus généralement dans les maladies résultant d'une diminution ou d'une perturbation des réponses immunitaires naturelles de l'organisme animal ou humain.

En outre, l'activité que possèdent les dérivés de la présente invention sur la réponse anticorps aux globules rouges de mouton chez la souris permet de penser que ces dérivés, tout en étant utiles dans les mêmes indications thérapeutiques que les composés de la demande 0 190 058, agissent par un mode d'action à la fois différent et complémentaire de celui des dérivés de l'art antérieur, ce qui les rend particulièrement intéressants dans leur domaine d'utilisation thérapeutique.

L'invention a également pour objet les compositions pharmaceutiques contenant au moins un composé de formule générale I, ou un de ses sels d'addition à un acide ou à une base pharmaceutiquement acceptable, seul ou en combinaison avec un ou plusieurs excipients ou véhicules inertes non toxiques, pharmaceutiquement acceptables.

Parmi les compositions pharmaceutiques selon l'invention, ou pourra citer plus particulièrement celles qui conviennent pour l'administration parentérale, per ou transcutanée, nasale, rectale, perlinguale, oculaire ou respiratoire, et notamment les préparations injectables, les aérosols, les gouttes oculaires ou nasales, les comprimés, les comprimés sublinguaux, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 microgramme et 1 gramme par prise ou par application.

Les exemples suivants illustrent l'invention, et ne la limitent en aucune façon.

Les produits de départ sont connus de la littérature.

Les points de fusion indiqués sont mesurés selon la technique micro-Kofler. Les spectres de résonance magnétique nucléaire du $^{13}C$ ont été enregistrés en utilisant le TMS comme référence interne, ceux de $^{1}H$ RMN ont généralement été enregistrés en utilisant le CD $Cl_3$ comme solvant. Les spectres de masse sont réalisés selon la technique F.A.B.

**EXEMPLE 1 :**

**Cyclo (S) Thr - (S) Lys - (S) ABO - (S) Arg - OH**

Cyclo (S) Thr - OH = (Trans L) carboxy - 4 methyl - 5 oxo - 2 oxazolidine 1, 3 ou (S) Cyclo thréonine décrit par Y. STABINSKI et al. Int. J. Peptide Prot. Res. 1978, 12, 130-138.

STADE A :

tBoc (S) ABO – OH ou acide (tert. butoxycarbonyl - 2 aza - 2 bicyclo [2.2.2] octane) carboxylique - 3 - (3S)

Préparé en utilisant la méthode décrite dans la demande de brevet européen n° 0 190 058 (exemple n° 8 - stade A).

STADE B :

tBoc(S)ABO-(S)Arg($NO_2$)-OBzl

En utilisant la méthode de W. KÖNIG et R. GEIGER (Ber. 1970, 103, 788), coupler 0,05 mole de tBoc (S) ABO-OH obtenu au stade précédent avec 0,05 mole de (S) $N_\omega$ nitro arginate de benzyle ou (S) H - Arg ($NO_2$)

- OBzl en utilisant le diméthylformamide comme solvant.

On obtient le tBoc (S) ABO - (S) Arg($NO_2$) - OBzl avec un rendement de 98% sous forme d'une huile utilisée telle quelle dans le stade suivant.

Caractéristiques spectrales en I.R (solution CH $Cl_3$)

ν (NH (amide)) = 3400 $cm^{-1}$

ν (CO) = 1700 $cm^{-1}$(large)

STADE C :

(S) ABO - (S) Arg ($NO_2$) - OBzl

En utilisant la méthode de déprotection par l'acide trifluoracétique dans le chlorure de méthylène anhydre décrite par B. GUTTE et R.B. MERRIFIELD (J. Am. Chem. Soc., 1969, 91, 501), on obtient quantitativement, à partir de 0,0475 mole de tBoc (S) ABO - (S) Arg ($NO_2$) - OBzl préparé au stade précédent, le (S) ABO - (S) Arg ($NO_2$) - OBzl sous forme de trifluoroacétate (TFA), dont la pureté est vérifiée par chromatographie sur couche mince (solvant : $CH_2$ $Cl_2$ - MeOH : 9 - 1 ; RF = 0,19).

STADE D :

tBoc (S) lys (Z) - (S) ABO - (S) Arg ($NO_2$) - OBzl

En remplaçant dans le stade B précédent le tBoc (S) ABO – OH par la $N_\alpha$ - tert-butoxycarbonyl $N_\omega$ benloxycarbonyl (S) lysine (ou tBoc (S) Lys (Z) – OH ; 0,0475 mole) et le (S) H - Arg ($NO_2$) - OBzl par le (S) ABO - (S) Arg ($NO_2$) - OBzl, TFA (0,0475 mole) préparé au stade précédent, on obtient de la même façon le tBoc(S) Lys (Z) - (S) ABO - (S) Arg ($NO_2$) - OBzl, avec un rendement de 68%.

Principales caractéristiques $^{13}C$ RMN (DMSO$d_6$/TMS)

δen ppm

| | |
|---|---|
| $C_\alpha$ Lysine | = 50,2 |
| $C_\alpha$ ABO | = 59,6 |
| $C_\alpha$ Arg | = 51,8 |

STADE E :

(S) Lys (Z) - (S) ABO - (S) Arg ($NO_2$) - OBzl

En déprotégeant le tBoc (S) Lys (Z) - (S) ABO - (S) Arg ($NO_2$) - OBzl par l'acide trifluoracétique, comme indiqué dans le stade C précédent, on obtient quantitativement le (S) Lys (Z) - (S) ABO - (S) Arg ($NO_2$) - OBzl sous forme de trifluoracétate, dont la pureté est vérifiée par chromatographie sur couche mince (solvant : $CH_2$ $Cl_2$ / MeOH : 95/5)

STADE F :

Cyclo (S) Thr - (S) Lys (Z) - (S) ABO - (S) Arg ($NO_2$) - OBzl

En couplant, selon la technique indiquée au stade B précédent, le (S) Lys (Z) - (S) ABO - (S) Arg ($NO_2$) - OBzl, avec la (S) cyclo Thréonine (ou Cyclo (S) Thr - OH), on obtient de la même façon le Cyclo (S) Thr - (S) Lys (Z) - (S) ABO - (S) Arg ($NO_2$) - OBzl, qui est purifié par chromatographie sur silice (60 - 230 mesh) en utilisant comme éluant le mélange $CH_2$ $Cl_2$ - MeOH (96/4).

Principales caractéristiques $^{13}C$ RMN (DMSO$d_6$/TMS)

δ en ppm

| | |
|---|---|
| $C_\alpha$ Cyclo Thr | = 60,0 |
| $C_\alpha$ Lys | = 49,0 |
| $C_\alpha$ ABO | = 59,7 |
| $C_\alpha$ Arg | = 51,5 |

STADE G :

Cyclo (S) Thr - (S) Lys - (S) ABO - (S) Arg - OH

Soumettre 0,0012 mole de Cyclo (S) Thr - (S) Lys (Z) - (S) ABO - (S) Arg ($NO_2$) - OBzl obtenu au stade précédent, à une hydrogénation catalytique dans 80 ml d'acide acétique, sous une pression d'hydrogène de 3 bars en présence de 400 mg de charbon palladié à 10%. Après évaporation du solvant sous pression réduite,

le résidu amorphe est repris par 5 ml d'eau distillée, séparé par microfiltration et lyophilisé.

On obtient le monoacétate de Cyclo (S) Thr - (S) Lys - (S) ABO - (S) Arg - OH (rendement 90%)

Caractéristiques spectrales en :

Infra-rouge :

ν (CO) : 1750 $cm^{-1}$

Spectrométrie de masse :

Spectre FAB + Kv (7 Kv) ion moléculaire protoné $[M + H]^+$ à M/z = 567 ($C_{25} H_{42} N_8 O_7$ ; PM = 566)

**EXEMPLE 2 :**

**Cyclo (S) Thr - (S) Lys - (S) THIQ - (S) Arg - OH**

STADE A :

tBoc (S) THIQ - OH ou acide (tert - butoxycarbonyl - 2 tétrahydro - 1, 2, 3, 4 isoquinoléine) - carboxylique - 3 - (3S)

Préparé selon la méthode décrite dans l'exemple 1, stade A de la demande de brevet européen 0 190 058 en remplaçant l'acide perhydroindole carboxylique - 2 (2S, 3aS, 7aS) par l'acide tétrahydro - 1.2.3.4 isoquinoléine carboxylique - 3 - ou (3S) THIQ - OH.

STADE B :

tBoc (S) THIQ - (S) Arg ($NO_2$) - $OCH_3$

En utilisant le (S) $N_\omega$ Nitro arginate de méthyle ou (S) H - Arg ($NO_2$) - $OCH_3$ et le tBoc (S) THIQ - OH obtenu au stade précédent on obtient selon la méthode de l'exemple 1, stade B le tBoc (S) THIQ - (S) Arg ($NO_2$) - $OCH_3$ sous forme d'une huile dont la pureté est vérifiée par chromatographie sur couche mince (solvant acétate d'éthyle ; Rf = 0,2)

Caractéristiques spectrales en IR :

ν (CO) = 1740 $cm^{-1}$

STADE C :

(S) THIQ - (S) Arg ($NO_2$) - $OCH_3$

En remplaçant dans le stade C de l'exemple 1 le tBoc (S) ABO - (S) Arg ($NO_2$) - OBzl par le tBoc (S) THIQ - (S) Arg ($NO_2$) - $OCH_3$ obtenu au stade précédent, on obtient de la même façon le (S) THIQ - (S) Arg ($NO_2$) - $OCH_3$ sous forme de trifluoracétate.

Caractéristiques spectrales en RMN ($^1H$)

1,7 ppm : 4H ($CH_2$)

3,2 ppm : 4H ($CH_2N$, $CH_2$ - Ø)

3,6 ppm : 3H ($COOCH_3$)

4,3 ppm : 4H (N - CH - CO, N - $CH_2$ - Ø)

7,3 ppm : 4H (phényl)

7,4 à 10,0 ppm : Protons échangeables.

STADE D :

tBoc (S) Lys (Z) - (S) THIQ - (S) Arg ($NO_2$) - $OCH_3$

En remplaçant dans le stade D de l'exemple 1 le (S) ABO - (S) Arg ($NO_2$) - OBzl, TFA par le (S) THIQ - (S) Arg ($NO_2$) - $OCH_3$, TFA obtenu au stade précédent on obtient le tBoc (S) Lys (Z) - (S) THIQ - (S) Arg ($NO_2$) - $OCH_3$ purifié par chromatographie sur gel silice (éluant : acétate d'éthyle, Rf : 0,16).

Rendement : 66%

STADE E :

(S) Lys (Z) - (S) THIQ - (S) Arg ($NO_2$) - $OCH_3$

Selon la méthode utilisée dans l'exemple 1, stade C on obtient à partir du tBoc (S) Lys (Z)-(S) THIQ- (S) Arg ($NO_2$) - $OCH_3$, le (S) Lys (Z) - (S) THIQ - (S) Arg ($NO_2$) - $OCH_3$ sous forme de trifluoracétate

Chromatographie sur couche mince (solvant : $CH_2Cl_2$ - MeOH : 90 - 10 ; Rf : 0,13)

STADE F :

Cyclo (S) Thr - (S) Lys (Z) - (S) THIQ - (S) Arg ($NO_2$) - $OCH_3$

En remplaçant dans le stade F de l'exemple 1 le (S) Lys (Z) - (S) ABO - (S) Arg ($NO_2$) - OBzl, TFA par le (S) Lys (Z) - (S) THIQ - (S) Arg ($NO_2$) - $OCH_3$, TFA on obtient le Cyclo (S) Thr - (S) Lys (Z) - (S) THIQ - (S) Arg ($NO_2$) - $OCH_3$ sous forme d'une huile épaisse utilisée telle quelle au stade suivant.

STADE G :

Cyclo (S) Thr - (S) Lys (Z) - (S) THIQ - (S) Arg ($NO_2$) - OH

Dissoudre 0,001 mole de Cyclo (S) Thr - (S) Lys (Z) - (S) THIQ - (S) Arg ($NO_2$) - $OCH_3$ obtenu au stade précédent dans 20 cm³ de méthanol, ajouter 10 cm³ de soude 0,1 N et maintenir 24 heures à température ambiante. Concentrer sous pression réduite, reprendre par 30 cm³ d'eau et neutraliser par addition de 10 cm³ d'acide chlorhydrique 0,1 N. Filtrer le précipité, laver à l'eau puis au chlorure de méthylène et sécher.

Rendement : 78%

Caractéristiques spectrales :

Spectrométrie de masse :

| FAB $^+$ | m/z | FAB $^-$ | m/z |
|---|---|---|---|
| $[M + K]^+$ | = 806 | $[M - 2H + Na]^-$ | = 788 |
| $[M - H + 2Na]^+$ | = 812 | $[M - H]^-$ | = 766 |
| $[M + Na]^+$ | = 790 | $[M - H - H_2O]^-$ | = 748 |
| $[M + H]^+$ | = 768 | $[M - H - CO_2]^-$ | = 722 |
| | | $[M - H - H_2NNO_2]^-$ | = 704 |

STADE H :

Cyclo (S) Thr - (S) Lys - (S) THIQ - (S) Arg - OH

Dissoudre 0,0005 mole de Cyclo (S) Thr - (S) Lys (Z) - (S) THIQ - (S) Arg ($NO_2$) - OH obtenu au stade précédent dans 50 cm³ d'acide acétique. Soumettre à une hydrogénation catalytique sous une pression d'hydrogène de 3 kg/cm² en présence de 200 mg de charbon palladié à 10 %. Après évaporation du solvant sous pression réduite, le résidu est repris par 5 cm³ d'eau distillée, séparé par microfiltration et lyophilisé. On obtient le mono acétate de Cyclo (S) Thr - (S) Lys - (S) THIQ - (S) Arg - OH.

Rendement : 98%

Caractéristiques spectrales :

Spectrométrie de masse :

| FAB $^+$ | m/z | FAB $^-$ | m/z |
|---|---|---|---|
| $[M_1 + H]^+$ | = 589 | $[M_1 - H]^-$ | = 587 |
| $[M_1 + Na]^+$ | = 611 | $[M_1 - H - CO_2]^-$ | = 543 |

**EXEMPLE 3 :**

**Cyclo (S) Thr - (S) Lys - PHII - (S) Arg - OH**

En remplaçant dans l'exemple 2, stade A, l'acide tétrahydro - 1.2.3.4 isoquinoléine carboxylique - 3 - (3S) ou (S) THIQ - OH par l'acide perhydroisoindole carboxylique - 1 ou PHII - OH, on obtient successivement :

tBoc PHII - OH
tBoc PHII - (S) Arg $(NO_2)$ - $OCH_3$
PHII - (S) Arg $(NO_2)$ - $OCH_3$, TFA
tBoc (S) Lys (Z) - PHII - (S) Arg $(NO_2)$ - $OCH_3$
(S) Lys (Z) - PHII - (S) Arg $(NO_2)$ - $OCH_3$, TFA
Cyclo (S) Thr - (S) Lys (Z) - PHII - (S) Arg $(NO_2)$ - $OCH_3$
Cyclo (S) Thr - (S) Lys (Z) - PHII (S) Arg $(NO_2)$ - OH
Cyclo (S) Thr - (S) Lys - PHII - (S) Arg - OH
lyophilisé sous forme de mono acétate
Caractéristiques spectrales :
Spectrométrie de masse :

| FAB $^+$ | m/z | FAB $^-$ | m/z |
|---|---|---|---|
| $[M_1 + H]^+$ | = 581 | $[M_1 - H]^-$ | = 579 |
| 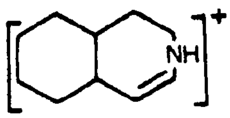 | = 124 | $[M_1 - H - CO_2]^-$ | = 535 |

**EXEMPLE 4 :**

**Cyclo (2S, 3R) AHPA - (S) Lys - (S) ABO - (S) Arg - OH**

Le cyclo (2S, 3R) AHPA - OH ou acide oxo - 2 benzyl - 4 oxazolidinyl - 1,3 carboxylique -5- (4R, 5S) décrit par H.UMEZAWA ; M. OHNO, - brevet européen n° 0 156 279 a été préparé selon la méthode de Y. STABINSKY et al. (Int. J. Peptide Prot. Res,. 1978, 12, 130-138) à partir du Z (2S, 3R) AHPA - OH ou acide hydroxy - 2 benzyloxycarbonylamino - 3 phényl - 4 butanoïque (2S, 3R) décrit par T. TAKITA et al. J. Med. Chem., 1977, 20, 510-515.

En remplaçant dans l'exemple 1, stade F le Cyclo (S) Thr - OH par le cyclo (2S, 3R) AHPA - OH on obtient successivement :

– Cyclo (2S, 3R) AHPA - (S) Lys (Z) - (S) ABO - (S) Arg $(NO_2)$ - OBzl
– Cyclo (2S, 3R) AHPA - (S) Lys - (S) ABO - (S) Arg - OH

lyophilisé sous forme de mono acétate.
Caractéristiques spectrales :
en IR : vs (C = O(oxazolidinone)) : 1760 $cm^{-1}$
en spectrométrie de masse : spectre FAB$^+$
ion moléculaire protoné $[M + H]^+$ à M/Z : 643

**EXEMPLE 5**

**[(4R) Méthyl - 4 oxo - 2 oxazolidinyl - 1,3 carbonyl - 5] - (S) Lys - (S) ABO - (S) Arg - OH**

En remplaçant dans l'exemple 4 le Z(2S, 3R) AHPA - OH par l'acide hydroxy - 2 benzyloxycarbonylamino - 3 butanoïque - (3R) décrit par T. TAKITA et al (J. Med. Chem., 1977, 20, 510-515), on obtient successivement:

– Acide oxo - 2 méthyl - 4 oxazolidinyl - 1,3 carboxylique - 5 - (4R) décrit par Y. SHIMOHIGASHI et al., Bull. Chem. Soc. Jpn., 1979, 52, (3), 949-950.
– [(4R) Méthyl - 4 oxo - 2 oxazolidinyl - 1,3 carbonyl - 5] - (S) Lys (Z) - (S) ABO - (S) Arg $(NO_2)$ OBzl
– [(4R) Méthyl - 4 oxo - 2 oxazolidinyl - 1,3 carbonyl - 5] - (S) Lys - (S) ABO - (S) Arg - OH

lyophilisé sous forme de mono acétate
Caractéristiques spectrales :
en IR : vs(CO(oxazolidinone)) : 1750 $cm^{-1}$
en spectrométrie de masse m/z : spectre FAB$^+$
$[M + H]^+ = 567$
$[M + Na]^+ = 589$
$[M - H + 2Na]^+ = 611$

**EXEMPLE 6 :**

**Cyclo GABOB - (S) Lys - (S) ABO - (S) Arg - OH**

STADE A :

N-Z GABOB - OH ou acide hydroxy - 3 benzyloxycarbonylamino - 4 butanoïque

Prépare selon la méthode de BERGMANN et ZERVAS (Ber, 1932 65,, 1192) à partir de l'acide hydroxy - 3 amino - 4 butanoïque (GABOB - OH).

STADE B :

Cyclo GABOB - OH ou carboxyméthyl - 5 oxo - 2 oxazolidine - 1,3

En utilisant la méthode décrite par Y. STABINSKY et al (Int. J. Peptide Prot. Res., 1978, 12, 130-138), on obtient le cyclo GABOB - OH à partir du NZ GABOB - OH préparé au stade précédent.

STADE C :

Cyclo GABOB - (S) Lys - (S) ABO - (S) Arg - OH

En remplaçant dans l'exemple 1, stade F la Cyclo (S) Thr par le cyclo GABOB - OH on obtient successivement :

– cyclo GABOB - (S) Lys (Z) - (S) ABO - (S) Arg ($NO_2$) - OBzl

– cyclo GABOB - (S) Lys - (S) ABO - (S) Arg - OH

lyophilisé sous forme de mono acétate.

**EXEMPLE 7 :**

**Cyclo Stat - (S) Lys - (S) ABO - (S) Arg - OH**

En remplaçant dans l'exemple 6, stade A l'acide hydroxy - 3 amino - 4 butanoïque ou GABOB OH par l'acide hydroxy - 3 amino - 4 méthyl - 6 heptanoïque ou statine (Stat - OH), on obtient successivement :

– Stade A : N - Z - Stat - OH ou acide hydroxy - 3 benzyloxycarbonylamino - 4 méthyl - 6 heptanoïque.

– Stade B : Cyclo Stat - OH ou oxo - 2 isobutyl - 4 carboxyméthyl - 5 oxazolidine - 1,3.

– Stade C : Cyclo Stat - (S) Lys - (S) ABO - (S) Arg - OH

lyophilisé sous forme de mono acétate.

**EXEMPLE 8 :**

**TZC - (S) Lys - (S) ABO - (S) Arg - OH**

En remplaçant dans l'exemple 1, stade F la (S) cyclo thréonine par l'acide (thiazolidinone - 2) carboxylique - 4 (TZC - OH), on obtient successivement :

– TZC - (S) Lys (Z) - (S) ABO - (S) Arg ($NO_2$) - OBzl

– TZC - (S) Lys - (S) ABO - (S) Arg - OH

lyophilisé sous forme de mono acétate.

Caractéristiques spectrales :

Spectrométrie de masse m/z

Spectre FAB+ : $[M + H]^+$ : 569

Spectre FAB- : $[M - H]^-$ : 567 $[M - H - H_2S]^-$ : 533

**EXEMPLE 9 :**

**Cyclo (S) Thr - (S) Lys - (S) PHI - (S) Arg - OH**

En remplaçant dans l'exemple 1, stade B, le tBoc (S) ABO - OH par l'acide (tert. butoxycarbonyl - 1 perhydroindole) carboxylique - 2 (2S, 3a S, 7a S) ou tBoc (S) PHI OH préparé comme indiqué dans la demande de brevet européen n° 0 190 058 (exemple 1 - stade A) et en procédant ensuite comme indiqué dans l'exemple

1 de la présente invention, du stade B au stade G, on obtient le dérivé :

Cyclo (S) Thr- (S) Lys - (S) PHI - (S) Arg - OH lyophilisé sous forme de mono acétate.

**EXEMPLE 10 :**

**DMT - (S) Lys - (S) ABO - (S) Arg - OH**

En remplaçant dans l'exemple 1, stade F la cyclothréonine par la diméthyl - 5,5 carboxy - 4 thiazolidinone - 2 ou DMT - OH préparée selon F.P DOYLE, D.O HOLLAND, P. MAMALIS et A. NORMAN (J.C.S 1958, 4605-4614), on obtient successivement :

DMT - (S) Lys (Z) - (S) ABO - (S) Arg (NO2) - OBzl

DMT - (S) Lys - (S) ABO - (S) Arg - OH

lyophilisé sous forme de mono acétate.

**EXEMPLES 11 à 13**

En opérant comme indiqué dans l'exemple 1, stade B, mais en remplaçant l'acide (tert butoxycarbonyl - 2 aza - 2 bicyclo [2.2.2] octane) carboxylique - 3 (3S) ou tBoc (S) ABO - OH par l'acide (tert butoxycarbonyl - 2 isoindoline) carboxylique - 1 - (1S) ou tBoc I.S.I. - OH ou par l'acide (tert butoxycarbonyl - 2 aza - 2 bicyclo[2.2.1] heptane) carboxylique - 3 ou tBoc ABH - OH ou par l'acide (tert butoxycarbonyl - 2 tétrahydro 1, 2, 3, 4 bétacarboline) carboxylique - 3 (3S) ou tBoc (S) THC - OH, on obtient les composés suivants :

Exemple 11 : Cyclo (S) Thr - (S) Lys - (S) ISI - (S) Arg - OH lyophilisé sous forme de mono acétate.

Exemple 12 : Cyclo (S) Thr - (S) Lys - ABH - (S) Arg - OH lyophilisé sous forme de mono acétate.

Exemple 13 : Cyclo (S) Thr - (S) Lys - (S) THC - (S) Arg - OH lyophilisé sous forme de mono acétate.

**EXEMPLES 14 et 15 :**

En opérant comme indiqué dans l'exemple 9, et en remplaçant le tBoc (S) PHI - OH par l'acide (t. butoxycarbonyl - 2 perhydroisoquinoléine) carboxylique - 3 ou tBoc PHIQ - OH ou par l'acide (t. butoxycarbonyl - 1 perhydrocyclopenta [b] pyrrole) carboxylique - 2 ou t.Boc PCP - OH, on obtient les composés suivants :

Exemple 14 : Cyclo (S) Thr - (S) Lys - PHIQ - (S) Arg - OH lyophilisé sous forme de mono acétate.

Exemple 15 : Cyclo (S) Thr - (S) Lys - PCP - (S) Arg - OH lyophilisé sous forme de mono acétate.

**EXEMPLES 16, 17 et 18 :**

En remplaçant dans l'exemple 4, le cyclo (2S, 3R) AHPA - OH par :

– l'acide oxo - 2 phényl - 4 oxazolidine - 1,3 carboxylique - 5 ou PH cyclo AHPA - OH

– l'acide oxo - 2 (thiényl - 2) - 4 oxazolidine - 1,3 carboxylique - 5 ou Thi cyclo AHPA - OH

– l'acide oxo - 2 (hydroxy - 3 phényl) - 4 oxazolidine - 1,3 carboxylique - 5 ou OHPh cyclo AHPA - OH

on obtient les produits suivants :

Exemple 16 : PH cyclo AHPA - (S) Lys - (S) ABO - (S) Arg - OH lyophilisé sous forme de mono acétate.

Exemple 17 : Thi cyclo AHPA - (S) Lys - (S) ABO - (S) Arg - OH lyophilisé sous forme de mono acétate

Exemple 18 : OHPh cyclo AHPA - (S) Lys - (S) ABO - (S) Arg - OH lyophilisé sous forme de mono acétate.

**EXEMPLE 19 :** Promotion de l'activité N.K.

Le composés selon l'invention ont été testés pour leur pouvoir promoteur de l'activité "Tueuse spontanée" ("Natural killer"). Les cellules dotées de ce pouvoir forment la première ligne de défense de l'organisme vis-à-vis de l'envahissement septique, viral ou tumoral.

Pour apprécier leur pouvoir stimulant, des composés selon l'invention ont été étudiés selon la technique REYNOLDS, et coll. 1981, (J. Immunol. 127, 282).

Les composés sont injectés par voie intraveineuse à la dose de 20 à 50 µg/kg à des souris de souche B6D2F1.

Trois jours après le traitement, les animaux sont sacrifiés, leur rate prélevée et dissociée en ses cellules constituantes qui sont ensemencées en culture en présence de cellules tumorales YAC-1 préalablement marquées au chrome radioactif. A l'issue de l'incubation, le pouvoir destructeur des composés de l'invention est mesuré par la quantité de chrome libérée.

A titre d'exemple, le composé de l'exemple 1, à la dose de 25 µg/kg, induit par rapport au témoin, une augmentation de la libération de chrome de 15%, identique à celle provoquée par le composé de l'exemple 8 de la demande de brevet européen n° 0 190 058, alors que la tuftsine à la dose de 40 µg/kg ne provoque qu'une libération de 10%.

**EXEMPLE 20 :** Inhibition de croissance du mélanome B 16.

Les mélanomes sont des tumeurs cancéreuses sensibles à la réaction du système immunitaire du malade. Ils constituent donc un modèle de choix pour apprécier toute stimulation de défense antitumorale.

Le composé selon l'exemple 9 s'est, par exemple, montré capable de ralentir de 45% la croissance du mélanome B 16 de la souris, lorsque le produit est administré à raison de 20 µg/kg, 3 fois par semaine par voie intrapéritonéale. Dans les mêmes conditions le pourcentage obtenu avec l'exemple 1 de la demande de brevet européen n° 0 190 058 n'est que de 40% et la tuftsine s'est montrée incapable de favoriser le ralentissement de croissance de la tumeur greffée.

**EXEMPLE 21 :** Augmentation de la résistance des animaux à l'infection.

Certaines souches bactériennes pathogènes sont capables de tuer l'hôte sain chez qui elles sont inoculées. C'est le cas, par exemple, de Klebsiella pneumoniae, agent responsable de la pneumonie (Parent, M. ; et coll. Proc. Natl. Acad. Sci.USA, 1978, 75, n°7, 3395).

Le composé de l'exemple 1, de façon identique au composé de l'exemple 8 de la demande de brevet européen n° 0 190 058, par exemple, est capable à la dose de 60 µg par animal de protéger de la mort par infection toutes les souris Swiss, femelles, de 20 à 25 g, auxquelles la souche Klebsiella pneumoniae 7823 est inoculée par voie IP, lorsqu'il est administré 48 heures avant l'infection. Dans les mêmes conditions, la tuftsine n'a été capable de sauver que 20% des animaux.

**EXEMPLE 22 :** Mesure de la réponse anticorps.

Les composés selon l'invention ont été testés pour leur pouvoir d'augmenter la réponse anticorps aux globules rouges de mouton chez la souris.

La réponse anticorps est mesurée in vitro selon la technique de JERNE (Science, 1963, 140, 405).

Des souris C 57 Bl/6 mâles, de poids moyen 23 g, ont été traitées par voie intraveineuse par les composés selon l'invention 48 heures avant l'inoculation intrapéritonéale de globules rouges de mouton. Cinq jours plus tard, la production d'anticorps spécifiques dirigés contre les globules rouges de mouton est détectée dans une suspension de cellules de la rate de ces animaux.

A titre d'exemple, le composé selon l'exemple 1 s'est montré capable, lorsque administré à la dose de 1 mg/kg d'augmenter de 50% la réponse anticorps aux globules rouges de mouton chez la souris.

A titre de comparaison, le dérivé de Y. STABINSKI et al, appelé [O = C$\psi$Thr] tuftsine n'a été capable dans les mêmes conditions, d'augmenter cette réponse anticorps que d'une valeur de 35%. Le dérivé correspondant de la demande de brevet européen n° 0 190 058, exemple 8, s'est révélé inactif sur ce test.

**EXEMPLE 23 :** Test de phagocytose non spécifique.

Les composés selon l'invention ont été testés pour leur pouvoir d'augmenter la phagocytose non spécifique.

Ce test consiste à évaluer, après traitement en cytométrie de flux, la capacité des polynucléaires contenus dans le sang total de chien à phagocyter des billes en latex de 1,8 µm de diamètre d'une part en l'absence (témoin), d'autre part en la présence d'un composé de l'invention.

A titre d'exemple le composé selon l'exemple 1 s'est montré capable d'augmenter de 19% par rapport au témoin, la capacité de phagocytose non spécifique des polynucléaires alors que la tuftsine, dans les mêmes conditions, ne donne pas de réponse significative.

**EXEMPLE 24 :** Hypersensibilité retardée à l'oxazolone.

Ce test consiste à sensibiliser un animal à l'oxazolone ; sept jours plus tard on effectue un test d'hypersensibilité retardée d'une part en l'absence (témoin), d'autre part après administration d'un composé selon l'invention et l'on effectue dans les deux cas la pesée de l'oreille de l'animal sacrifié. L'oreille est le siège d'une réaction inflammatoire et son poids est fonction de l'intensité de cette réaction. On constate qu'après un traitement par le composé selon l'exemple 1, l'augmentation de poids de l'oreille des animaux en expérience est supérieure de 11% au lot des animaux témoins. La tuftsine ne donne pas de réponse significative dans ce test.

**EXEMPLE 25 :** Compositions pharmaceutiques.

Soluté injectable

| | | | |
|---|---|---|---|
| Cyclo (S) Thr - (S) Lys - (S) PHI - (S) Arg - OH : | | 0,050 | g |
| eau pour préparation injectable : | | 2 | $cm^3$ |

Crème dermique

| | | | |
|---|---|---|---|
| Cyclo [(S) Thr] - (S) Lys - (S) ABO - (S) Arg - OH | | 5 | g |
| polypropylèneglycol | | 25 | g |
| vaseline blanche | | 10 | g |
| alcool à 95° | | 10 | g |
| eau purifiée | q.s.p. | 100 | g |

## Revendications

1. Composés de formule générale

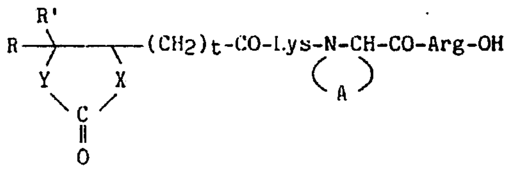

I

dans laquelle

R représente

– un atome d'hydrogène

– un radical alcoyle comportant de 1 à 4 atomes de carbone, en chaîne droite ou ramifiée,

– un radical phényle ou thiényle éventuellement substitué par un groupement hydroxy, amino, mercapto, méthylthio, ou alcoyle, ou inférieur

– un radical benzyle,

R' représente un atome d'hydrogène ou un groupement alcoyle comportant de 1 à 4 atomes de carbone en chaîne droite ou ramifiée,

X représente un atome d'oxygène ou un groupement NH,

Y représente un atome d'oxygène ou de soufre lorsque X représente un groupement NH ou Y représente un groupement NH lorsque X représente un atome d'oxygène,

t représente 0 ou 1,

Lys et Arg représentent respectivement les restes lysile et arginyle engagés dans des liaisons peptidiques,

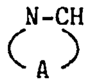

représente

1) une structure bicyclique de formule

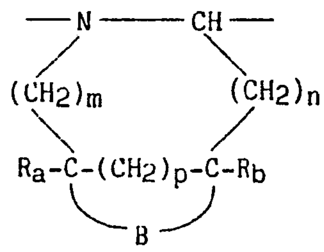

où

– m est égal à 1 ou zéro,

– n et p représentent zéro, 1 ou 2,

– $R_a$ et $R_b$ représentent un atome d'hydrogène ou peuvent former ensemble une liaison directe quand p =0,

– B représente une chaîne alkylène $(CH_2)_q$ où q est égal à 2, 3 ou 4 ou bien une structure insaturée $(-CH=CH-)_2$ quand p = 0 et $R_a$ et $R_b$ forment ensemble une liaison, avec la réserve que la somme de m, n et q est un nombre entier compris entre 3 et 6, ou

2) la tétrahydro - 1, 2, 3, 4 - bétacarboline, leurs énantiomères, épimères et diastéréoisomères, ainsi que leurs sels d'addition à un acide ou une base pharmaceutiquement acceptable.

2. Composés selon la revendication 1 dans lesquels la structure cyclique

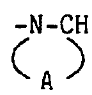

représente l'indoline, l'isoindoline, la tétrahydroyuinoléine, la tétrahydroisoquinoléine, le perhydroindole, le perhydroisoindole, la perhydroquinoléine, la perhydroisoquinoléine, le perhydrocyclopenta [b] pyrrole, l'aza - 2 bicyclo [2.2.2] octane, l'aza - 2 bicyclo [2.2.1] heptane, la tétrahydro - 1, 2, 3, 4 bétacarboline, leurs énantiomères, épimères et diastéréoisomeres, ainsi que leurs sels d'addition à un acide ou une base pharmaceutiquement acceptable.

3. Composés selon l'une quelconque des revendications 1 ou 2 dans lesquels la structure cyclique

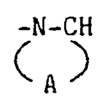

représente le perhydroindole ou l'azabicyclo [2 2 2] octane, leurs énantiomères, épimères et diastéréoisomeres, ainsi que leurs sels d'addition à un acide ou une base pharmaceutiquement acceptable.

4. Le Cyclo (S) Thr - (S) Lys - (S) AB0 - (S) Arg - OH et ses sels d'addition à un acide ou une base pharmaceutiquement acceptable.

5. Le Cyclo (S) Thr - (S) Lys - (S) PHI - (S) Arg - OH et ses sels d'addition à un acide ou une base pharmaceutiquement acceptable.

6. Le Cyclo (S) Thr - (S) Lys - (S) THIQ - (S) Arg - OH et ses sels d'addition à un acide ou une base pharmaceutiquement acceptable.

7. Le Cyclo (S) Thr - (S) Lys - PHII - (S) Arg - OH ses énantiomères et diastéréoisomères ainsi que ses sels d'addition à un acide ou une base pharmaceutiquement acceptable.

8. Le Cyclo (2S, 3R) AHPA - (S) Lys - (S) AB0 - (S) Arg - OH et ses sels d'addition à un acide ou une base pharmaceutiquement acceptable.

9. Le [(4R) Méthyl - 4 oxo - 2 oxazolidinyl -1,3 carbonyl - S] - (S) Lys - (S) AB0 - (S) Arg - OH, ses énantiomères et leurs sels d'addition à un acide ou une base pharmaceutiquement acceptable.

10. Le Cyclo GABOB - (S) Lys - (S) AB0 - (S) Arg - OH, ses ènantiomères, et leurs sels d'addition a un acide ou une base pharmaceutiquement acceptable.

11. Procédé de préparation des composés de formule 1 caractérisé en ce que l'on condense un dérivé de formule II

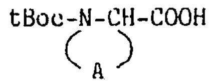

II

dans lequel tBoc représente le radical tertiobutoxycarbonyle et A avec les atomes de carbone et d'azote auxquels il est attaché, a les mêmes significations que dans la formule I, avec le $N_{\omega}$ - nitroarginate de méthyle (H - Arg ($NO_2$) $OCH_3$) ou de benzyle (H - Arg ($NO_2$) $OCH_2$ $C_6H_5$) pour obtenir un dérivé de formule III

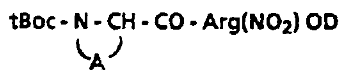

III

dans lequel A, avec les atomes de carbone et d'azote auxquels il est attaché, a les mêmes significations que dans la formule I, et dans lequel D représente un radical méthyle ou benzyle que l'on déprotège ensuite par l'acide trifluoroacétique en un dérivé de formule IV

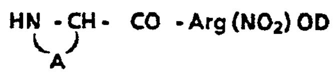

IV

dans lequel A, avec les atomes de carbone et d'azote auxquels il est attaché, a les mêmes significations que dans le formule I, et dans lequel D à la même signification que dans la formule III qui est ensuite condensé avec la $N_{\alpha}$ - tertiobutoxycarbonyl $N_{\omega}$ benzyloxycarbonyl lysine ou (tBoc) Lys (Z) pour obtenir un composé de formule V

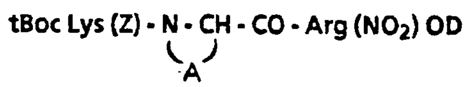

V

dans lequel A, avec les atomes de carbone et d'azote auxquels il est attaché, a les mêmes significations que dans la formule I et D la même signification que dans la formule III. qui est soumis à l'action de l'acide trifluoroacétique et converti en un dérivé de formule VI

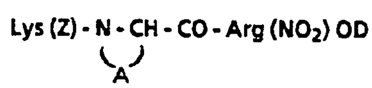

VI

dans lequel A, avec les atomes de carbone et d'azote auxquels il est attaché, a les mêmes significations que dans la formule I et D la même signification dans la formule III que l'on condense avec un dérivé de formule VII

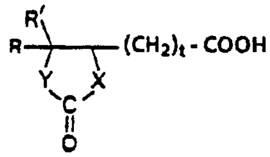

VII

dans lequel
R, R', X, Y et t ont la même signification que dans la formule I pour conduire à un dérivé de formule VIII

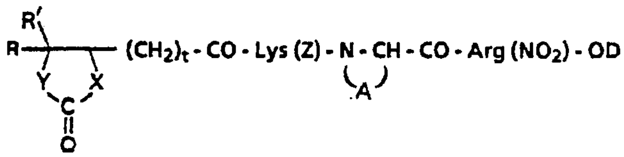

VIII

dans lequel A, avec les atomes de carbone et d'azote auxquels il est attaché, R, R', X, Y et t ont la même signification que dans la formule I et D la même signification que dans la formule III qui peut-être soit
- quand D représente $CH_3$, déprotégé par saponification puis soumis à une hydrogénation catalytique
- quand D représente le groupement benzyle, soumis à hydrogénation catalytique pour conduire à un dérivé de formule I que l'on peut si l'on désire
- soit salifier par un acide ou une base pharmaceutiquement acceptable,
- soit séparer en ses isomères, puis, si nécessaire, salifier par un acide ou une base pharmaceutiquement acceptable.

12. Composés de structure générale VIII selon la revendication 11 utiles pour la préparation des composés selon la revendication 1.

13. Composition pharmaceutique contenant comme principe actif au moins un composé selon la revendication 1 à 10 en combinaison avec un ou plusieurs excipients ou véhicules inertes non-toxiques, pharmaceutiquement acceptable.

14. Composition pharmaceutique selon la revendication 13 utile dans le traitement des maladies résultant d'une diminution ou d'une perturbation des défenses immunitaires naturelles de l'organisme.

**Claims**

1. Compounds of the general formula :

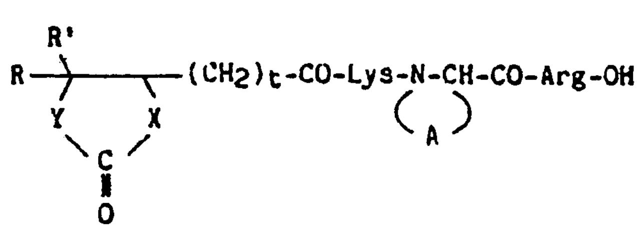

I

in which
R represents :
- a hydrogen atom,
- a straight-chained or branched alkyl radical containing from 1 to 4 carbon atoms,
- a phenyl or thienyl radical optionally substituted by a hydroxy, amino, mercapto, methylthio or lower alkyl group, or

– a benzyl radical,

R′ represents a hydrogen atom or a straight-chained or branched alkyl group containing from 1 to 4 carbon atoms,

X represents an oxygen atom or an NH group,

Y represents an oxygen or sulphur atom when X represents an NH group, or Y represents an NH group when X represents an oxygen atom,

t represents 0 or 1,

Lys and Arg represent, respectively, the lysine and arginine residues linked by peptide bonds,

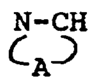

represents

1) a bicyclic structure of the formula :

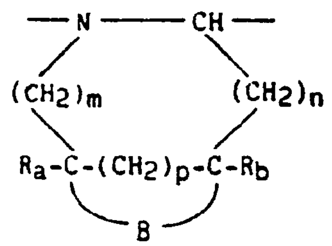

wherein

– m is 1 or 0,

– n and p represent 0, 1 or 2,

– $R_a$ and $R_b$ represent a hydrogen atom, or together they can form a direct bond when p = 0,

– B represents a $(CH_2)_q$ alkylene chain, wherein q is 2, 3 or 4, or alternatively an unsaturated $(-CH=CH-)_2$ structure when p = 0 and $R_a$ and $R_b$ together form a bond, with the proviso that the sum of m, n, p and q is an integer of from 3 to 6, or

2) 1, 2, 3, 4-tetrahydrobetacarboline, their enantiomers, epimers and diastereoisomers, and also their addition salts with a pharmaceutically acceptable acid or base.

2. Compounds according to claim 1 in which the cyclic structure

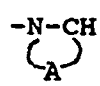

represents indoline, isoindoline, tetrahydroquinoline, tetrahydroisoquinoline, perhydroindole, perhydroisoindole, perhydroquinoline, perhydroisoquinoline, perhydrocyclopenta[b]pyrrole, 2-azabicyclo[2.2.2]octane, 2-azabicyclo[2.2.1]heptane or 1, 2, 3, 4-tetrahydrobetacarboline, their enantiomers, epimers and diastereoisomers, and also their addition salts with a pharmaceutically acceptable acid or base.

3. Compounds according to any one of claims 1 and 2 in which the cyclic structure

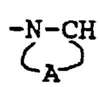

represents perhydroindole or azabicyclo[2.2.2]octane, their enantiomers, epimers and diastereoisomers, and also their addition salts with a pharmaceutically acceptable acid or base.

4. Cyclo (S) Thr - (S) Lys - (S) ABO - (S) Arg - OH and its addition salts with a pharmaceutically acceptable acid or base.

5. Cyclo (S) Thr - (S) Lys - (S) PHI - (S) Arg - OH and its addition salts with a pharmaceutically acceptable acid or base.

6. Cyclo (S) Thr - (S) Lys - (S) THIQ - (S) Arg - OH and its addition salts with a pharmaceutically acceptable

acid or base.

7. Cyclo (S) Thr - (S) Lys - PHII - (S) Arg - OH, its enantiomers and diastereoisomers, and also its addition salts with a pharmaceutically acceptable acid or base.

8. Cyclo (2S, 3R) AHPA - (S) Lys - (S) ABO - (S) Arg - OH and its addition salts with a pharmaceutically acceptable acid or base.

9. [(4R)-4-methyl-2-oxo-1, 3-oxazolidinyl-5-carbonyl]-(S) Lys - (S) AB0 - (S) Arg - OH, its enantiomers, and their addition salts with a pharmaceutically acceptable acid or base.

10. Cyclo GABOB - (S) Lys - (S) AB0 - (S) Arg - OH, its enantiomers, and their addition salts with a pharmaceutically acceptable acid or base.

11. Process for the preparation of the compounds of the formula I, characterised in that a derivative of the formula II

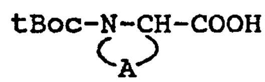

II,

in which tBoc represents the tert.-butoxycarbonyl radical and A, together with the carbon and nitrogen atoms to which it is bonded, has the same meanings as in formula I, is condensed with the $N_\omega$-nitroarginate of (H-Arg($NO_2$)$OCH_3$)-methyl or (H-Arg($NO_2$)$OCH_2C_6H_5$)-benzyl to give a derivative of the formula III

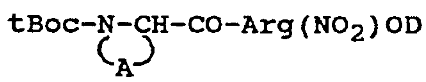

III,

in which A, together with the carbon and nitrogen atoms to which it is bonded, has the same meanings as in formula I and in which D represents a methyl or benzyl radical, which is then deprotected by means of trifluoroacetic acid to give a derivative of the formula IV

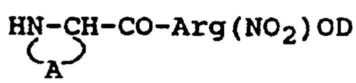

IV,

in which A, together with the carbon and nitrogen atoms to which it is bonded, has the same meanings as in formula I and in which D has the same meaning as in formula III, which is then condensed with $N_\alpha$-tert.-butoxycarbonyl-$N_\omega$benzyloxycarbonyl-lysine or with (tBoc)Lys(Z) to give a compound of the formula V

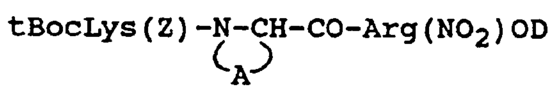

V,

in which A, together with the carbon and nitrogen atoms to which it is bonded, has the same meanings as in formula I and D has the same meaning as in formula III, which is subjjected to the action of trifluoroacetic acid and converted into a derivative of the formula VI

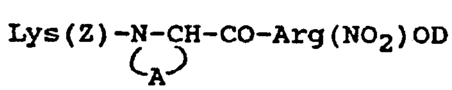

VI,

in which A, together with the carbon and nitrogen atoms to which it is bonded, has the same meanings as in formula I and D has the same meaning as in formula III, which is condensed with a derivative of the formula VII

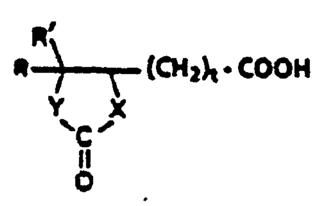

VII,

in which

R, R′, X, Y and t have the same meaning as in formula I, to give a derivative of the formula VIII

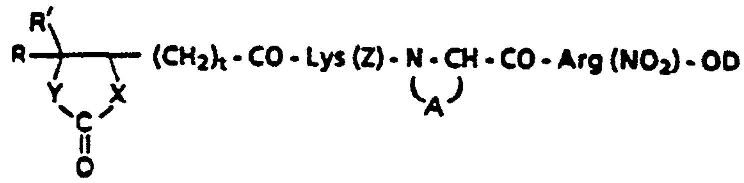

VIII,

in which A, together with the carbon and nitrogen atoms to which it is bonded, R, R′, X, Y and t have the same meaning as in formula I and D has the same meaning as in formula III, which may either

– when D represents $CH_3$, be deprotected by hydrolysis and then subjected to catalytic hydrogenation, or

– when D represents the benzyl group, be subjected to catalytic hydrogenation, to give a derivative of the formula I which, if desired, may

– either be converted into a salt with a pharmaceutically acceptable acid or base,

– or be separated into its isomers and then, if necessary, be converted into a salt with a pharmaceutically acceptable acid or base.

12. Compounds of the general structure VIII according to claim 11 for use in the preparation of the compounds according to claim 1.

13. Pharmaceutical composition containing as active ingredient at least one compound according to claims I to 10 in combination with one or more inert, non-toxic, pharmaceutically acceptable excipients or vehicles.

14. Pharmaceutical composition according to claim 13 for use in the treatment of diseases resulting from a reduction or disturbance in the natural immune defences of the organism.

**Ansprüche**

1. Verbindungen der allgemeinen Formel (I)

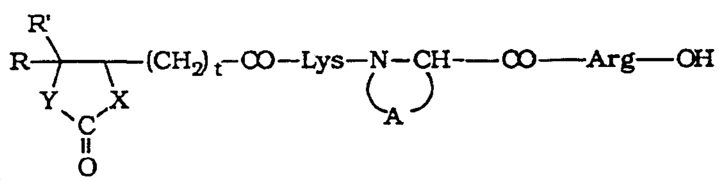

I

in der

R

– ein Wasserstoffatom,

– eine geradkettige oder verzweigte Alkylgruppe mit 1 bis 4 Kohlenstoffatomen,

– eine Phenyl- oder Thienyl-Gruppe, die gegebenenfalls durch eine Hydroxylgruppe, eine Aminogruppe, eine Mercaptogruppe, eine Methylthiogruppe oder eine niedrigmolekulare Alkylgruppe substituiert ist, oder

– eine Benzylgruppe,

R′ ein Wasserstoffatom oder eine geradkettige oder verzweigte Alkylgruppe mit 1 bis 4 Kohlenstoffatomen,

X ein Sauerstoffatom oder eine NH-Gruppe,

Y ein Sauerstoffatom oder ein Schwefelatom, wennXeine NH-Gruppe darstellt, oder eine NH-Gruppe, wenn X ein Sauerstoffatom darstellt,

t 0 oder 1,

Lys beziehungsweise Arg in Peptidbindungen eingebundene Lisyl- beziehungsweise Arginylreste, der Rest der Formel

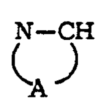

1) einen bicyclischen Rest der Formel

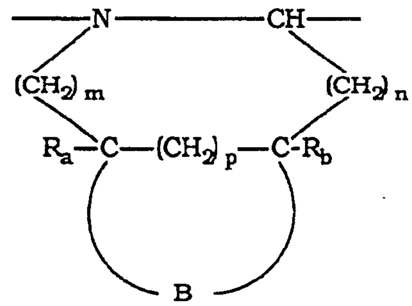

in der

– m 1 oder 0,

– n 0, 1 oder 2,

– $R_a$und $R_b$ Wasserstoffatome oder gemeinsam, wenn p = 0 ist, eine direke Bindung,

– B eine Alkylenkette der Formel $(CH_2)_q$, worin q 2, 3 oder 4 darstellt, oder

– eine ungesättigte Kette der Formel $(-CH = CH-)_2$, wenn p = 0 ist und $R_a$ und $R_b$ gemeinsam eine Bindung darstellen, mit der Maßgabe, daß die Summe von m, n, p und q eine ganze Zahl zwischen 3 und 6 ist, oder

2) einen 1, 2, 3, 4-Tetrahydro-betacarbolin-Rest bedeuten, ihre Enantiomère, Epimère und Diastereoisomere sowie ihre Additionssalzepharmazeutisch annehmbaren Säure oder Base.

2. Verbindungen nach Anspruch 1, worin der cyclische Rest der Formel

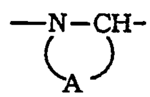

einen Indolin-, Isouindolin Tetrahydroisochinolin-, Tetrahydrochinolin-, Tetrahydroisochlin-, Perhydroindol-, Perhydroisoindol-, Perhydrochinoollin-, Perhydrosochinolin-, Perhydrocyclopenta[b]pyrol-, 2-Aza-bicyclo[2.2.2]octan, 2-Aza-bicyclo[2.2. 1]heptan oder 1, 2, 3, 4-Tetrahydro-betacarbolin-Rest bedeutet, ihre Enantlomere, Epimere und Diastereoisomere sowie ihre Additionssalze mit einer pharmazeutisch annehmbaren Säure oder Base.

3. Verbindungen nach einem der Anspüche 1 oder 2, worin der cyclische Rest der Formel

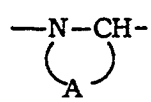

den Perhydroindol- oder Azabicyclo[2.2.2]octan-Rest bedeutet, ihre Enantiomere, Epimere und Diastereoisomere sowie ihre Additionssalze mit einer pharmazeutisch annehmbaren Säure oder Base.

4. Cyclo-(S)-THr-(S)Lys-(S)-AB0-(S)-Arg-OH und dessen Additionssalze mit einer pharmazeutlsch annehmbaren Säure oder Base.

5. Cyclo-(S) -THr-(S)Lys-(S)PHI-(S)Arg-OH und dessen Additionssalze mit einer pharmazeutisch annehmbaren Säure oder Base.

6. Cyclo-(S)-THr-(S)Lys-(S)THIQ-(S)Arg-OH und dessen Additionssalze mit einer pharmazeutisch annehmbaren Säure oder Base.

7. Cyclo-(S) -THr- (S)Lys-PHII- (S)Arg-OH, dessen Enantiomere und Diastereoisomere sowie dessen Additionssalze mit einer pharmazeutisch annehmbaren Säure oder Base.

8. Cyclo-(2S,3RAHPA-(S)Lys-(S)ABO-(S)Arg-OH und dessen Additionssalze mit einer pharmazeutisch annehmbaren Säure oder Base.

9. [(4R)-4-Methyl-2-oxo- 1,3-oxazolidinyl-5-carbonyl]-(S)Lys-(S)ABO-(S)ArgOH, dessen Enantiomere und dessen Additionssalze mit einer azlharmazeutlsch annehmbaren Säure oder Base.

10. Cyclo-GABOB-(S)Lys-(S)ABO-(S)Arg-OH, dessen Enantiomere und dessen Additionssalze mit einer pharmazeutisch annehmbaren Säure oder Base.

11. Verfahren zur Herstellung der Verbindungen der Formel I, **dadurch gekennzeichnet,** daß man ein Derivat der Formel II

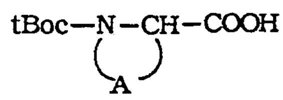 II

in der tBoc den Tertbutoxycarbonylrest bedeutet und A mit dem Kohlenstoffatom und dem Stickstoffatom, an die es gebunden ist, die gleichen Bedeutungen besitzt. wie sie bezüglich der Formel I angegeben sind, mit $N_\omega$ -Nitroarginimethylester (H-Arg($NO_2$)$OCH_3$ oder $N_\omega$ -Nitroarginibenzylester (H-Arg($NO_2$)$OCH_2C_6H_5$) umsetzt zur Bildung einer Verbindung der Formel III

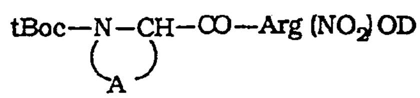 III

in der A zusammen mit dem Kohlenstoffatom und dem Stickstoffatom, an die es gebunden is die gleichen Bedeutungen besitzt, wie sie für die Formel I angegeben sind, und D eine Methyl- oder Benzyl-Gruppe bedeutet, von welchen man anschließend mit Trifluoressigsäure die Schutzgruppen abspaltet zur Bildung eines Derivats der Formel IV

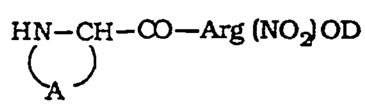 IV

in der Azusammen mit dem Kohlenstoffatom und dem Stickstoffatom, an die es gebunden ist, die gleichen Bedeutungen besitzt, wie sie für die Formel I angegeben sind, und worin D die gleichen Bedeutungen besitzt, wie sie bezüglich der Formel III angegeben sind, das anschließend mit $N_\alpha$-tert. -Butoxycarbonyl-$N_\omega$-benzyloxycarbonyl-lysin oder (tBoc)-Lys(Z) kondensiert wird zur Bildung einer Verbindung der Formel V

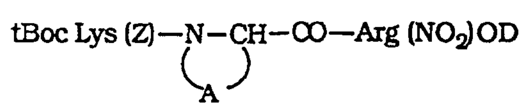 V

in der Azusammen mit dem Kohlenstoffatom und dem Stickstoffatom, an die es gebunden ist, die gleichen Bedeutungen besitzt, wie sie für die Formel I angegeben sind, und D die gleichen Bedeutungen besitzt, wie sie für die Formel III angegeben sind, die anschließend mit Trifluoressigsäure behandelt und in ein Derivat der Formel VI umgewandelt wird

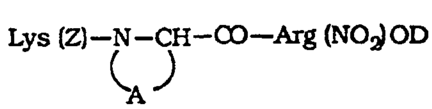 VI

in der Azusammen mit dem Kohlenstoffatom und dem Stickstoffatom, an die es gebunden ist, die gleichen Bedeutungen besitzt, wie sie bezüglich der Formel I angegeben sind und D die bezüglich der Formel III angegebenen Bedeutungen aufweist, das man mit einem Derivat der Formel VII

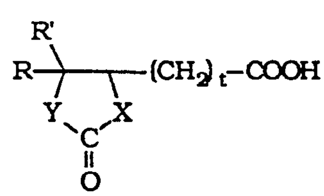 VII

in der R, R', X, Y und t die bezüglich der Formel I angegebenen Bedeutungen besitzen, kondensiert zur Bildung eines Derivats der Formel VIII

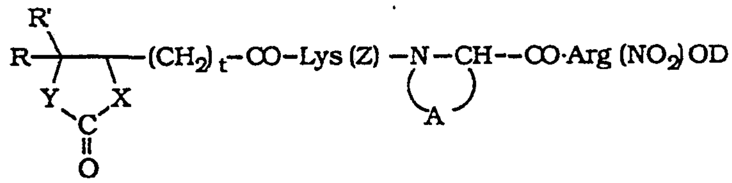

VIII

in der Azusammen mit dem Kohlenstoffatom und dem Stickstoffatom, an die esgebunden ist, R, R', X, Y und t die gleichen Bedeutungen besitzen, wie sie für die Formel I angegeben sind und D die gleichen Bedeutungen besitzt, wie sie für die Formel III angegeben sind, welches man

- entweder, wenn D eine $CH_3$-Gruppe bedeutet, durch Verseifen von der Schutzgruppe befreit und dann eier katalytischen Hydrierung unterwirft,
- oder, wenn D eine Benzylgruppe darstellt, einer katalytischen Hydrierung unterwirft, zur Bildung eines Derivats der Formel I, das man gewünschtenfalls
- entweder mit einer pharmazeutisch annehmbaren Säure oder Base in ein Salz überführen,
- oder in seine Isomeren auftrennen und erforderlichenfalls mit eher pharmazeutisch annehmbaren Säure oder Base in ein Salz überführen kann.

12. Verbindungen der allgemeinen Formel VIII nach Anspruch 11 für die Herstellung der Verbindungen nach Anspruch 1.

13. Pharmazeutische Zubereitung enthaltend als Wirkstoffmindestens eine Verbindung nach den Ansprüchen 1 bis 10 in Kombination mit ehem oder mehreren inerten, nichttoxischen, pharmazeutisch annehmbaren Bindemitteln oder Trägermaterialien.

14. Pharmazeutische Zubereitung nach Anspruch 13 zur Behandlung von Erkrankungen, die zu einer Verminderung oder Störung des natürlichen Immunsystems des Organismus führen.